# EUROPÄISCHE PATENTSCHRIFT

(11) **EP 3 883 496 B1**
(45) Veröffentlichungstag und Bekanntmachung des Hinweises auf die Patenterteilung: **15.01.2025**
(21) Anmeldenummer: 19801289.0
(22) Anmeldetag: 07.11.2019
(51) Int. Cl.: A61F 2/16, A61F 9/00, A61L 27/28

(54) **OPHTHALMOLOGISCHES IMPLANTAT MIT EINEM WIRKSTOFFABGABESYSTEM UND VERFAHREN ZUM HERSTELLEN EINES SOLCHEN OPHTHALMOLOGISCHEN IMPLANTATS**
OPHTHALMOLOGICAL IMPLANT COMPRISING AN ACTIVE INGREDIENT RELEASE SYSTEM AND METHOD FOR PRODUCING AN OPHTHALMOLOGICAL IMPLANT OF THIS TYPE
IMPLANT OPHTALMOLOGIQUE COMPORTANT UN SYSTÈME DE DISTRIBUTION DE PRINCIPE ACTIF ET PROCÉDÉ DE FABRICATION D'UN TEL IMPLANT OPHTALMOLOGIQUE

(30) Priorität: 22.11.2018 DE 102018129478
(43) Veröffentlichungstag der Anmeldung: 29.09.2021
(73) Patentinhaber: Carl Zeiss Meditec AG, 07745 Jena (DE)
(72) Erfinder: THALLER, Michael, 10585 Berlin (DE)
(74) Vertreter: Hofstetter, Schurack & Partner
(86) Internationale Anmeldenummer: PCT/EP2019/080503
(87) Internationale Veröffentlichungsnummer: WO 2020/104200

(56) Entgegenhaltungen:
- EP-B1- 1 071 482
- WO-A1-2008/113043
- WO-A1-95/03783
- DE-A1- 19 700 082

## Beschreibung

### Technisches Gebiet

Die Erfindung betrifft ein ophthalmologisches Implantat mit einem Wirkstoffabgabesystem, welches dazu ausgebildet ist, im implantierten Zustand des ophthalmologischen Implantats wenigstens einen pharmakologischen Wirkstoff abzugeben. Die Erfindung betrifft weiterhin ein Verfahren zum Herstellen eines solchen ophthalmologischen Implantats.

### Stand der Technik

Als Fibrose (fachsprachlich auch Fibrosis) wird eine krankhafte Vermehrung des Bindegewebes in menschlichen und tierischen Geweben bezeichnet, dessen Hauptbestandteil Kollagenfasern sind. Dabei wird das Gewebe des betroffenen Organs verhärtet. Es entstehen narbige Veränderungen, die im fortgeschrittenen Stadium zur Einschränkung der jeweiligen Organfunktion führen. Bei der Verwendung von ophthalmologischen Implantaten, beispielsweise von Intraokularlinsen (lOLs) können aufgrund von Wechselwirkungen zwischen dem Implantat und angrenzendem biologischem Gewebe verschiedene Komplikationen auftreten, von denen insbesondere solche Fibrosen problematisch sind. Beispielsweise tritt nach Katarakt-Operationen in manchen Fällen eine sogenannte posteriore Kapselopazifikation (posterior capsule opacification, PCO, Cataracta secundaria) auf. Bei PCO handelt es sich um eine postoperative Trübung der Linsenkapsel nach der chirurgischen Extraktion einer natürlichen Linse. Die verbleibenden Linsenepithelzellen (E-Zellen) in der äquatorialen Region des Kapselsacks sind mitotisch aktiv und können zu Fibroblasten transformieren. Diese lösen dann eine Art Wundheilung aus, wobei kollagenhaltiges Bindegewebe gebildet wird. Da manche Fibroplasten-Subtypen nicht nur an die Innenseite des Kapselsacks migrieren, sondern sich auch zusammenziehen können, kommt es zur Faltenbildung im Kapselsack. Die Trübung der Kapsel ist somit die Folge eines Wundheilungsprozesses und einer damit verbundenen Narbenbildung. Da die dadurch verursachte Linsentrübung andere Ursachen als die ursprüngliche Katarakt-Erkrankung besitzt, spricht man von einem "Nachstar" oder einem "sekundären Katarakt". Ein klinisch signifikanter Nachstar kann bei den Betroffenen zu einer Minderung der Sehschärfe, der Farbwahrnehmung und des Kontrastsehens sowie zu einer erhöhten Blendung führen.

Beim sekundären Katarakt handelt es sich um eine häufige Komplikation nach extrakapsulären Kataraktoperation (ECCE) und der anschließenden Implantation einer Intraokularlinse (IOL) in den Kapselsack. Ohne die Implantation einer IOL in den leeren Kapselsack ist das Risiko eines Nachstars sogar noch erheblich erhöht, da in diesem Fall eine ungehinderte Zellmigration zur hinteren Oberfläche des Kapselsackes möglich ist.

Die Inzidenz eines sekundären Katarakts erhöht sich nach einem operativen Eingriff mit der Zeit. Eine Meta-Analyse der Fälle von Cataracta secundaria für alle bestehenden Arten von IOLs zeigte postoperativ eine ungefähre durchschnittliche Zunahme von 12% nach einem Jahr und eine ungefähre durchschnittliche Zunahme von 30% nach fünf Jahren. Ein entscheidender Faktor scheint hierbei auch das Alter des betreffenden Patienten zu sein, so dass damit gerechnet werden muss, dass fast alle behandelten Kinder und Jugendlichen nach einer gewissen Zeit unter postoperativem sekundären Katarakt leiden könnten.

Eine weitere häufige postoperative Komplikation der Kataraktoperation ist die Augenentzündung. Dies wird durch die Wunde in der Hornhaut verursacht, die durch den Hornhautschnitt entsteht, der für den Ersatz der natürlichen Linse durch ein künstliches Implantat erforderlich ist. Dieses physische Trauma kann eine entzündliche Reaktion auslösen, indem es entzündliche Mediatoren wie Prostaglandine und Leukotriene freisetzt, die anschließend eine Immunantwort im Augeninneren auslösen. Kurzfristig kann dies zu Schmerzen und Beschwerden beim Patienten führen. Bei unsachgemäßer Behandlung kann eine anhaltende Entzündung zu schwerwiegenden Nebenwirkungen wie posteriorer Synechie, Uveitis und sekundärem Glaukom führen. Es ist daher wichtig, die postoperative Operation so gut wie möglich zu gestalten.

Das postoperative Entzündungsmanagement wird typischerweise mit Medikamenten durchgeführt. Derzeit gibt es zwei anerkannte Wirkstoffklassen, eine auf Basis von Corticosteroiden und eine auf Basis der so genannten "nicht-steroidalen Entzündungshemmer" ("non-steroidal anti-inflammatory drugs", NSAIDS). Die steroidalen Medikamente gelten allgemein als wirksamer, haben aber Nebenwirkungen wie z.B. einen Anstieg des Augeninnendrucks, während die NSAIDS weniger Komplikationen verursachen, dafür aber generell weniger wirksam sind, wobei die Untersuchungen zu diesem Themenfeld noch nicht abgeschlossen sind.

Ein gewisses Problem stellt auch die Notwendigkeit der sogenannten Patienten-Compliance dar. Entzündungshemmende Medikamente müssen in der Regel für 4-6 Wochen verschrieben und während dieser Zeit vom Patienten selbst über Augentropfen verabreicht werden. Es kommt jedoch vor, dass sich Patienten nicht an ihr Medikamentenregime halten. Dies kann beispielsweise daran liegen, dass sie die Notwendigkeit der Behandlung nicht erkennen, sich die Kosten für das Medikament nicht leisten wollen oder können, körperlich nicht in der Lage sind, die Medikamente selbst richtig anzuwenden oder die Verabreichung vergessen. Diese mangelnde Einhaltung des Medikamentenplans beobachtet man oft bei mehr als der Hälfte der Patienten. Selbst bei regelmäßigen Untersuchungen ist diese mangelnde Einhaltung des Medikamentenplans für einen Arzt aber oft nicht erkennbar. Außerdem gibt es einige Bereiche, in denen die Patienten beispielsweise aufgrund einer langen Anreisedauer nicht regelmäßig zur Untersuchung in eine Praxis kommen. Diese Versäumnisse können zu Problemen bei der postoperativen Behandlung und zu Unannehmlichkeiten für den Patienten führen. Daher ist es sehr wünschenswert, auf eine aktive Beteiligung des Patienten bei der Umsetzung eines postoperativen Medikamentenplans möglichst zu verzichten und einen Weg zu finden, eine korrekte Medikamentenverabreichung zu gewährleisten. Dies sollte vorzugsweise so erfolgen, dass eine solche Lösung in den normalen Ablauf einer Augenoperation integriert wird oder werden kann.

Aus der WO 2008/113043 A1 ist ein ophthalmologisches Implantat bekannt, welches als Intraokularlinse mit einem optischen Teil und zwei haptischen Teilen ausgebildet ist. An wenigstens einem der haptischen Teile ist ein Wirkstoffabgabesystem angeordnet, um im implantierten Zustand des Implantats einen pharmakologischen Wirkstoff abgeben zu können.

Die WO 1995/03783 A1 beschreibt eine intraokulare Polymervorrichtung sowie ein Verfahren zu ihrer Herstellung und Verwendung, wobei die Polymervorrichtung ein Polymer und mindestens ein zytotoxisches Mittel, insbesondere ein Immuntoxin zur Vorbeugung und Behandlung von sekundären Katarakten, umfasst.

Die DE 197 00 082 A1 offenbart ein Verfahren zur Herstellung von zellproliferationsinhibierenden, kovalent fixierten Beschichtungen auf der Oberfläche von Substraten für den medizinischen Bereich. Dabei wird eine strahleninduzierte Pfropfpolymerisation durchgeführt.

Als nachteilig an dem bekannten ophthalmologischen Implantat ist der Umstand anzusehen, dass die Menge an abgebbarem Wirkstoff vergleichsweise gering ist, da nur die dünnen haptischen Teile verwendet werden, es sei denn, das Abgabesystem selbst ist relativ sperrig. Dies würde aber die erforderliche Inzisionsgröße bei der Implantation erhöhen und die Linse für die chirurgische Anwendung weniger sicher machen. Zudem würde hierdurch eine Beeinträchtigung der Funktionalität der haptischen Teile und damit auch des optischen Teils wahrscheinlicher.

### Darstellung der Erfindung

Aufgabe der vorliegenden Erfindung ist es, ein ophthalmologisches Implantat zu schaffen, welches eine verbesserte und länger andauernde Wirkstoffabgabe ermöglicht. Eine weitere Aufgabe der Erfindung ist es, die Herstellung eines solchen ophthalmologischen Implantats zu ermöglichen.

Die Aufgaben werden erfindungsgemäß durch ein ophthalmologisches Implantat mit den Merkmalen des Patentanspruchs 1 sowie durch ein Verfahren mit den Merkmalen des Patentanspruchs 9 gelöst. Vorteilhafte Ausgestaltungen mit zweckmäßigen Ausbildungen der Erfindung sind in den jeweiligen Unteransprüchen angegeben, wobei vorteilhafte Ausgestaltungen jedes Erfindungsaspekts als vorteilhafte Ausgestaltungen des jeweils anderen Erfindungsaspekts anzusehen sind.

Ein erster Aspekt der Erfindung betrifft ein ophthalmologisches Implantat gemäß Anspruch 1 mit einem Wirkstoffabgabesystem, welches dazu ausgebildet ist, im implantierten Zustand des ophthalmologischen Implantats wenigstens einen pharmakologischen Wirkstoff abzugeben. Erfindungsgemäß ist es vorgesehen, dass der Wirkstoff in im Hydrogel verteilten Polymernanopartikeln vorliegt. Eine verbesserte und länger andauernde Wirkstoffabgabe ist dadurch ermöglicht, dass das Wirkstoffabgabesystem wenigstens ein Hydrogel als Matrix umfasst, wobei das Hydrogel eine Schicht auf einem optischen Teil des Implantats bildet, kovalent an den optischen Teil gebunden und mit dem wenigstens einen Wirkstoff beladen ist. Mit anderen Worten ist es vorgesehen, dass das Wirkstoffabgabesystem als Beschichtung auf dem optischen Teil des Implantats angeordnet und durch kovalente Anbindung des Hydrogels an den optischen Teil immobilisiert ist. Das Hydrogel fungiert dabei generell als Matrix für den abzugebenden Wirkstoff, mit dem das Hydrogel beladen ist. Hierdurch ist es im Unterschied zu einer Anordnung an einem haptischen Teil möglich, eine wesentlich größere Fläche des Implantats für die Anordnung des Wirkstoffabgabesystems mit einer entsprechend höheren Wirkstoffmenge zu nutzen, ohne Einschränkungen hinsichtlich des Raumbedarfs, der Implantierbarkeit und der Funktionalität des Implantats hinnehmen zu müssen. Da Hydrogele im hydratisierten Zustand definitionsgemäß eine vergleichsweise große Menge an Wasser enthalten bzw. bei Kontakt mit Wasser häufig ein Mehrfaches ihres eigenen Gewichts an Wasser aufnehmen können, ist ihr Brechungsindex im implantierten und hydratisierten Zustand mit dem der Augenumgebung vergleichbar. Daher sind Hydrogele für das menschliche und tierische Auge praktisch unsichtbar. Dies ermöglicht es, das Hydrogel als Matrix des Wirkstoffabgabesystems schichtförmig, das heißt flächig und nicht in Form von isolierten Partikeln oder dergleichen, auf die optische Oberfläche des Implantats aufzutragen, da es die optischen Eigenschaften des optischen Teils nicht beeinträchtigt. Darüber hinaus können auf diese Weise Implantate unabhängig von einem haptischen Teil, also beispielsweise Implantate mit nur einem haptischen Teil oder Implantate ohne haptische Teile, mit dem Wirkstoffabgabesystem ausgestattet werden. Wenn das Implantat ein haptisches Teil oder mehrere haptische Teile aufweist, kann es vorgesehen sein, dass der haptische Teil oder die haptischen Teile ohne Wirkstoffabgabesystem ausgebildet ist bzw. sind. Alternativ kann das Wirkstoffabgabesystem zusätzlich zum optischen Teil auch an einem oder mehreren haptischen Teilen ausgebildet sein, wobei dies in der Regel weniger bevorzugt ist. Das Wirkstoffabgabesystem kann dabei grundsätzlich auf einem oder mehreren Oberflächenbereichen des optischen Teils oder auf der gesamten Oberfläche des optischen Teils angeordnet sein. Beispielsweise kann das Hydrogel 1 %, 2 %, 3 %, 4 %, 5 %, 6 %, 7%, 8%, 9 %, 10 %, 11 %, 12 %, 13%, 14%, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 %, 35 %, 36 %, 37 %, 38 %, 39 %, 40 %, 41 %, 42 %, 43 %, 44 %, 45 %, 46 %, 47 %, 48 %, 49 %, 50 %, 51 %, 52 %, 53 %, 54 %, 55 %, 56 %, 57 %, 58 %, 59 %, 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % oder 100 % der Oberfläche des optischen Teils bedecken. Weiterhin kann das Wirkstoffabgabesystem eine einzige Schicht bilden oder mehrschichtig, das heißt aus zwei oder mehr Schichten gebildet sein, wobei in der Regel eine einzige Schicht bevorzugt ist. Im Fall mehrerer Schichten können diese jeweils die gleiche Zusammensetzung oder unterschiedliche Zusammensetzungen aufweisen, gleich oder unterschiedlich dick sein sowie gleiche oder unterschiedliche Wirkstoffe oder Wirkstoffkombinationen enthalten. Die zwei oder mehr Schichten können ebenfalls kovalent miteinander gekoppelt sein, um eine besonders zuverlässige Anbindung des gesamten Schichtsystems an den optischen Teil zu gewährleisten. Die Gesamtdicke der Hydrogel-Schicht(en) kann in bestimmten Anwendungen bis zu 200 µm, also beispielsweise 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, 20 µm, 21 µm, 22 µm, 23 µm, 24 µm, 25 µm, 26 µm, 27 µm, 28 µm, 29 µm, 30 µm, 31 µm, 32 µm, 33 µm, 34 µm, 35 µm, 36 µm, 37 µm, 38 µm, 39 µm, 40 µm, 41 µm, 42 µm, 43 µm, 44 µm, 45 µm, 46 µm, 47 µm, 48 µm, 49 µm, 50 µm, 51 µm, 52 µm, 53 µm, 54 µm, 55 µm, 56 µm, 57 µm, 58 µm, 59 µm, 60 µm, 61 µm, 62 µm, 63 µm, 64 µm, 65 µm, 66 µm, 67 µm, 68 µm, 69 µm, 70 µm, 71 µm, 72 µm, 73 µm, 74 µm, 75 µm, 76 µm, 77 µm, 78 µm, 79 µm, 80 µm, 81 µm, 82 µm, 83 µm, 84 µm, 85 µm, 86 µm, 87 µm, 88 µm, 89 µm, 90 µm, 91 µm, 92 µm, 93 µm, 94 µm, 95 µm, 96 µm, 97 µm, 98 µm, 99 µm, 100 µm, 101 µm, 102 µm, 103 µm, 104 µm, 105 µm, 106 µm, 107 µm, 108 µm, 109 µm, 110 µm, 111 µm, 112 µm, 113 µm, 114 µm, 115 µm, 116 µm, 117 µm, 118 µm, 119 µm, 120 µm, 121 µm, 122 µm, 123 µm, 124 µm, 125 µm, 126 µm, 127 µm, 128 µm, 129 µm, 130 µm, 131 µm, 132 µm, 133 µm, 134 µm, 135 µm, 136 µm, 137 µm, 138 µm, 139 µm, 140 µm, 141 µm, 142 µm, 143 µm, 144 µm, 145 µm, 146 µm, 147 µm, 148 µm, 149 µm, 150 µm, 151 µm, 152 µm, 153 µm, 154 µm, 155 µm, 156 µm, 157 µm, 158 µm, 159 µm, 160 µm, 161 µm, 162 µm, 163 µm, 164 µm, 165 µm, 166 µm, 167 µm, 168 µm, 169 µm, 170 µm, 171 µm, 172 µm, 173 µm, 174 µm, 175 µm, 176 µm, 177 µm, 178 µm, 179 µm, 180 µm, 181 µm, 182 µm, 183 µm, 184 µm, 185 µm, 186 µm, 187 µm, 188 µm, 189 µm, 190 µm, 191 µm, 192 µm, 193 µm, 194 µm, 195 µm, 196 µm, 197 µm, 198 µm, 199 µm oder 200 µm betragen. Grundsätzlich können aber auch größere Schichtdicken, beispielsweise 200 µm, 250 µm, 300 µm, 350 µm, 400 µm, 450 µm, 500 µm oder mehr, vorgesehen sein, wobei entsprechende Zwischenwerte als mitoffenbart anzusehen sind. Ein Wirkstoff im Sinne der vorliegenden Offenbarung kann generell ein pharmakologisch wirksamer Stoff, der gegebenenfalls in Form seines Salzes, als Konjugat etc. vorliegt, und/oder eine Vorläufersubstanz ("Prodrug") sein, die erst nach Metabolisierung aktiv wird. Die Polymernanopartikel können ihrerseits selbst aus einem Hydrogel bestehen. Hierdurch kann die Freisetzungsrate des Wirkstoffs besonders präzise eingestellt werden. Zusätzlich kann der Wirkstoff vor unerwünschten Reaktionen bei der Ausbildung des Hydrogel-Netzwerks geschützt werden. Generell sind "ein/eine" im Rahmen dieser Offenbarung als unbestimmte Artikel zu lesen, also ohne ausdrücklich gegenteilige Angabe immer auch als "mindestens ein/mindestens eine". Umgekehrt können "ein/eine" auch als "nur ein/nur eine" verstanden werden.

In einer vorteilhaften Ausgestaltung der Erfindung ist das Implantat als Intraokularlinse, insbesondere akkommodierende Intraokularlinse ausgebildet. Hierdurch können die erfindungsgemäß realisierbaren Vorteile im Rahmen unterschiedlicher Augenoperationen und Implantattypen realisiert werden. Dies gilt in besonderem Maße für ein als akkommodierende Intraokularlinse ausgebildetes Implantat, da bei einer solchen IOL Entzündungsreaktionen, Fibrosen und dergleichen die Akkomodationsfähigkeit der IOL beeinträchtigen oder vollständig verhindern könnten. Für eine chirurgische Insertion werden Intraokularlinsen üblicherweise gefaltet und mit einem Injektor in das Auge injiziert. Um eine mögliche Schädigung der Hornhaut zu begrenzen, ist es wünschenswert, die Schnittgröße soweit wie möglich zu reduzieren und die Injektionsspitzen des Injektors so klein wie möglich zu halten. Dies führt dazu, dass ein gewisser Kraftaufwand erforderlich ist, um die gefaltete IOL durch den Injektor zu drücken. Diese Kräfte werden in der Regel durch eine Gleitbeschichtung der Innenwand des Injektors reduziert. Da das erfindungsgemäße Implantat mit einem Hydrogel beschichtet ist, stellt das Medikamente freisetzende Hydrogel des Wirkstoffabgabesystems vorteilhaft eine zusätzliche Schmierung bereit und verbessert dadurch entweder den bestehenden Injektionsprozess oder kann die bisherige Gleitbeschichtung an der Injektorwand sogar vollständig ersetzen bzw. überflüssig machen.

In einer weiteren vorteilhaften Ausgestaltung der Erfindung ist es vorgesehen, dass das Hydrogel abbaubar, insbesondere biologisch abbaubar ist. Dies ermöglicht eine besonders gut kontrollierbare Freisetzung des wenigstens einen, im Hydrogel gelagerten Wirkstoffs, wodurch unerwünschte Abgabespitzen besonders zuverlässig verhindert und der Wirkstoff über einen Zeitraum von mehreren Wochen kontinuierlich abgegeben werden kann. Der Abbau des Hydrogels kann beispielsweise dadurch erfolgen, dass das Hydrogel hydrolysierbare Polymerbindungen und/oder hydrolysierbare chemische Bindungen zu den Wirkstoffmolekülen aufweist. Alternativ oder zusätzlich kann das Hydrogel enzymatisch abbaubare Polymerbindungen und/oder enzymatisch abbaubare Bindungen zu den Wirkstoffmolekülen aufweisen. Beispielsweise können Peptidbindungen vorgesehen sein, die nach der Implantation durch Peptidasen gespalten werden. Die Abgabe des Wirkstoffs kann dabei derart ausgestaltet sein, dass der Wirkstoff ausschließlich durch Abbau des Hydrogels freigesetzt werden kann, beispielsweise indem der Wirkstoff an das Hydrogel gebunden ist, und/oder indem der Wirkstoff im Gitter der Hydrogel-Matrix sterisch "gefangen" ist. Alternativ kann vorgesehen sein, dass der Wirkstoff zusätzlich zur Freisetzung durch Abbau des Hydrogels auch durch Diffusion aus dem Hydrogel heraus freigesetzt werden kann, wodurch generell eine höhere Freisetzungsrate möglich ist.

In einer weiteren vorteilhaften Ausgestaltung der Erfindung ist vorgesehen, dass das Hydrogel ausgewählt ist aus einer Gruppe, die Poly(N-isopropylacrylamid), Polyvinylalkohol, Polyethylenglycol, Polymilchsäure, Polyethylenimin, Cellulose, Celluloseether mit Methyl- und/oder Ethyl- und/oder Propylgruppen, insbesondere Hydroxypropylmethylcellulose, Hydroxyethylmethylcellulose und/oder Methylcellulose, Glykosaminoglykane, insbesondere Hyaluronsäure, Chondroitinsulfat, Dermatansulfat, Heparin, Heparansulfat, Keratansulfat, Alginsäure, Polymannuronsäure, Polyguluronsäure, Polyglucuronsäure, Amylose, Amylopektin, Callose, Chitosan, Polygalactomannan, Dextran, Xanthan, und/oder eine Mischung und/oder ein physiologisch akzeptables Salz davon umfasst. Hierdurch können die Eigenschaften des Hydrogels, beispielsweise seine Viskoelastizität und Abbaubarkeit, optimal an den jeweiligen Einsatz- und Verwendungszweck des Implantats angepasst werden. Hydrophile langkettige Polymere können beispielsweise durch einfache Vernetzungsreaktionen zu Hydrogelen umgewandelt werden. Aufgrund der großen Menge an hydrophilen Gruppen kann das Gel anschließend eine entsprechend große Menge an Wasser aufnehmen, oft ein Vielfaches seines Eigengewichts. Dieselben Wechselwirkungen können genutzt werden, um Wirkstoffmoleküle innerhalb des Hydrogelnetzwerks physikalisch einzufangen. Für eine langsame und lang anhaltende Freisetzung werden hydrophile und vergleichsweise große bzw. sterisch anspruchsvolle Wirkstoffmoleküle bevorzugt, da sie nicht so schnell aus dem Hydrogel diffundieren und die Wahrscheinlichkeit einer sogenannten Burst-Freisetzung eines Großteils der vorhandenen Wirkstoffmenge kurz nach der Implantation verringern. Kleine hydrophobe Wirkstoffe könnten aber auch in solchen Netzwerken erfolgreich eingeschlossen werden, zum Beispiel durch Copolymerisation mit anderen Monomeren oder durch Manipulation der Oberfläche des Polymers. Alternativ können solche Wirkstoffmoleküle über eine abbaubare Bindung kovalent mit dem Polymer verbunden werden. Die Abbaurate steuert dann die Freisetzung der Wirkstoffe anstelle der Diffusion dieser Moleküle.

Polysaccharide, die auch als Glykane bezeichnet werden, stellen generell eine Unterklasse der Kohlenhydrate dar und sind Vielfachzucker aus Monosaccharideinheiten (z. B. Glukose, Fruktose, Galactose usw.), welche eine Kette bilden. Jedes Monosaccharid, das auch als Einfachzucker bezeichnet wird, besteht aus einer Kette von Kohlenstoff-Atomen. Nach der Anzahl der Kohlenstoffatome spricht man von Triosen (3), Tetrosen (4), Pentosen (5), Hexosen (6), Heptosen (7) usw. Polysaccharide können nach Art ihrer Saccharideinheiten in Homoglykane mit nur einer Art Einfachzucker und Heteroglykane mit zwei oder mehr verschiedenen Arten von Einzelzuckern eingeteilt werden. Weiterhin können Polysaccharide unsubstituiert oder substituiert sein und eine oder mehrere Seitengruppen wie beispielsweise Hydroxy-, Carboxy-, Amino- oder Sulfatgruppen tragen.

In einer weiteren vorteilhaften Ausgestaltung der Erfindung ist vorgesehen, dass der wenigstens eine Wirkstoff über einen vorzugsweise biologisch abbaubaren Bindungstyp kovalent an das Hydrogel gebunden ist. Dies ermöglicht ebenfalls eine besonders gut kontrollierbare Freisetzung des an das Hydrogel gebundenen Wirkstoffs, wodurch unerwünschte Abgabespitzen besonders zuverlässig verhindert und der Wirkstoff über einen Zeitraum von mehreren Wochen kontinuierlich abgegeben werden kann. Die kontrollierte Freisetzung des Wirkstoffs kann dabei auch in Verbindung mit einem nicht- oder schwer-abbaubaren Hydrogel realisiert werden. Der Abbau der kovalenten Bindung und die Freisetzung des Wirkstoffs kann auch in diesem Fall beispielsweise dadurch erfolgen, dass der Wirkstoff hydrolysierbare chemische Bindungen zu dem Hydrogel-Netzwerk aufweist. Alternativ oder zusätzlich kann der Wirkstoff enzymatisch abbaubare Bindungen zum Hydrogel-Netzwerk aufweisen. Beispielsweise können Peptidbindungen vorgesehen sein, die nach der Implantation durch Peptidasen gespalten werden. Alternativ oder zusätzlich ist es vorgesehen, dass der Wirkstoff kovalent an ein Monomer und/oder Oligomer gebunden ist. Hierdurch kann der Raumbedarf des Wirkstoffs erhöht werden, so dass auch kleine Wirkstoffmoleküle durch sterische Hinderung nicht oder nur mit niedrigen Raten aus dem Hydrogel-Netzwerk diffundieren können. Vorzugsweise beeinträchtigt das Monomer bzw. Oligomer die pharmakologische Wirkung des Wirkstoffs nicht. Alternativ kann vorgesehen sein, dass die Bindung des Wirkstoffs an das Monomer/Oligomer abbaubar ist, so dass der Wirkstoff vor und/oder nach der Diffusion aus dem Hydrogel-Netzwerk in seiner physiologischen Wirkform vorliegt. Weiterhin kann es vorgesehen sein, dass der Wirkstoff verteilt im Hydrogel vorliegt. Dies kann dadurch erreicht werden, dass der Wirkstoff während der Polymerisation der Hydrogel-Edukte bzw.

Precursoren in der Reaktionsmischung vorliegt und in dem gebildeten Netzwerk "gefangen" wird und/oder dass das Hydrogel in eine vorzugsweise gesättigte Lösung des Wirkstoffs eingebracht wird, so dass der Wirkstoff in das Hydrogel eindiffundiert. Gemäß der Erfindung ist vorgesehen, dass der Wirkstoff in insbesondere biologisch abbaubaren, im Hydrogel verteilten Polymernanopartikeln vorliegt. Die Polymernanopartikel können ihrerseits selbst aus einem Hydrogel bestehen und sind biologisch abbaubar. Hierdurch kann die Freisetzungsrate des Wirkstoffs besonders präzise eingestellt werden. Zusätzlich kann der Wirkstoff vor unerwünschten Reaktionen bei der Ausbildung des Hydrogel-Netzwerks geschützt werden.

In einer weiteren vorteilhaften Ausgestaltung der Erfindung ist wenigstens ein pharmakologischer Wirkstoff kovalent auf zumindest einem Oberflächenbereich des Hydrogels gebunden. Hierdurch kann die gewünschte pharmakologische Wirkung unmittelbar an der Oberfläche des Implantats erzielt werden. Beispielsweise kann es sich bei dem Wirkstoff um einen Antikörper handeln, der unerwünschte Zielstrukturen, beispielsweise Entzündungsmediatoren, Fibrogene oder dergleichen, direkt an der Oberfläche des Implantats abfängt und bindet. Hierdurch können Beeinträchtigungen der optischen Eigenschaften des Implantats besonders zuverlässig verhindert werden.

In einer weiteren vorteilhaften Ausgestaltung der Erfindung ist vorgesehen, dass der wenigstens eine Wirkstoff ausgewählt ist aus einer Gruppe, die steroidale und nicht-steroidale Entzündungshemmer, insbesondere COX-2-Hemmer, Prostaglandine und/oder Prostamide, Antibiotika und Betablocker umfasst. Hierdurch kann die pharmakologische Wirkung des Implantats optimal an unterschiedliche zu erwartende körpereigene Reaktionen und potenzielle Komplikationen, beispielsweise an Entzündungsreaktionen und/oder PCO, angepasst werden.

In einer weiteren vorteilhaften Ausgestaltung der Erfindung ist das Implantat in einer gesättigten Lösung des wenigstens einen Wirkstoffs gelagert ist. Hierdurch kann insbesondere in Fällen, in denen der Wirkstoff nicht fest an das Hydrogel gebunden oder anderweitig im Hydrogel "gefangen" ist, sondern beispielsweise durch Diffusion abgegeben werden soll, sichergestellt werden, dass die Konzentration des Wirkstoffs im Wirkstoffabgabesystem während der Lagerung des Implantats konstant bleibt.

Ein zweiter Aspekt der Erfindung betrifft ein Verfahren gemäß Anspruch 9 zum Herstellen eines ophthalmologischen Implantats mit einem Wirkstoffabgabesystem, welches dazu ausgebildet ist, im implantierten Zustand des ophthalmologischen Implantats wenigstens einen pharmakologischen Wirkstoff abzugeben. Eine verbesserte und länger andauernde Wirkstoffabgabe ist dadurch ermöglicht, dass ein optischer Teil des Implantats mit wenigstens einem Hydrogel als Matrix des Wirkstoffabgabesystems beschichtet und das wenigstens eine Hydrogel kovalent an den optischen Teil gebunden wird, wobei das Hydrogel mit dem wenigstens einen Wirkstoff beladen wird. Erfindungsgemäß liegt der wenigstens eine Wirkstoff in Polymernanopartikeln vor, die im Hydrogel verteilt werden. Mit anderen Worten ist es vorgesehen, dass das Wirkstoffabgabesystem als Beschichtung auf dem optischen Teil des Implantats angeordnet und durch kovalente Anbindung des Hydrogels an den optischen Teil immobilisiert ist. Das Hydrogel fungiert dabei generell als Matrix für den abzugebenden Wirkstoff, mit dem das Hydrogel beladen ist. Hierdurch ist es im Unterschied zu einer Anordnung an einem haptischen Teil möglich, eine wesentlich größere Fläche des Implantats für die Anordnung des Wirkstoffabgabesystems mit einer entsprechend höheren Wirkstoffmenge zu nutzen, ohne Einschränkungen hinsichtlich des Raumbedarfs, der Implantierbarkeit und der Funktionalität des Implantats hinnehmen zu müssen. Da Hydrogele im hydratisierten Zustand definitionsgemäß eine vergleichsweise große Menge an Wasser enthalten bzw. bei Kontakt mit Wasser häufig ein Mehrfaches ihres eigenen Gewichts an Wasser aufnehmen können, ist ihr Brechungsindex im implantierten und hydratisierten Zustand mit dem der Augenumgebung vergleichbar. Daher sind Hydrogele für das menschliche und tierische Auge praktisch unsichtbar. Das Beladen des Hydrogels mit dem Wirkstoff oder der Wirkstoffkombination kann generell bereits vor dem Beschichten des optischen Teils mit dem Hydrogel, während des Beschichtens des optischen Teils mit dem Hydrogel und/oder nach dem kovalenten Anbinden des Hydrogels an den optischen Teil erfolgen. Weiterhin kann vorgesehen sein, dass das Hydrogel zunächst im teilweise oder vollständig dehydratisierten Zustand auf den optischen Teil aufgebracht, kovalent gebunden und anschließend durch Inkontaktbringen mit Wasser hydratisiert wird. Die kovalente Anbindung des Hydrogels kann mit jeder geeigneten chemischen Methode, beispielsweise durch sogenannte Klickchemie durchgeführt werden. Die Polymernanopartikel können ihrerseits selbst aus einem Hydrogel bestehen. Hierdurch kann die Freisetzungsrate des Wirkstoffs besonders präzise eingestellt werden. Zusätzlich kann der Wirkstoff vor unerwünschten Reaktionen bei der Ausbildung des Hydrogel-Netzwerks geschützt werden. Weitere Merkmale und Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts anzusehen sind. Umgekehrt sind vorteilhafte Ausgestaltungen des zweiten Erfindungsaspekts als vorteilhafte Ausgestaltungen des ersten Erfindungsaspekts anzusehen.

In einer vorteilhaften Ausgestaltung der Erfindung ist vorgesehen, dass das kovalente Binden des wenigstens einen Hydrogels an den optischen Teil des Implantats die Schritte Erzeugen von oberflächlichen Hydroxygruppen auf dem optischen Teil des Implantats, Pfropfpolymerisieren wenigstens einer reaktiven Silanverbindung, welche neben einer Silangruppe wenigstens eine weitere funktionelle Gruppe trägt, auf die oberflächlichen Hydroxygruppen des Implantats und kovalentes Binden des wenigstens einen Hydrogels an die weitere funktionelle Gruppe der aufgepfropften Silanverbindung umfasst. Das Erzeugen von oberflächlichen Hydroxygruppen auf dem optischen Teil des Implantats ist generell nur dann erforderlich, wenn der optische Teil von sich aus keine oder eine zu geringe Anzahl an freien Hydroxygruppen aufweist. Die Hydroxygruppen werden vorzugsweise durch Plasmaaktivierung erzeugt, wobei generell auch andere geeignete Techniken verwendet werden können. Als Silanverbindung eignen sich insbesondere Trialkoxysilane der allgemeinen Formel (I) 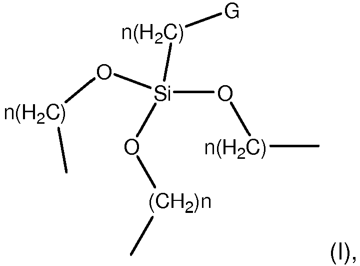 in der G die funktionelle Gruppe bezeichnet und die Parameter n unabhängig voneinander gewählt werden können, das heißt 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 oder mehr betragen können. G kann beispielsweise eine Aminogruppe oder eine Ethenylgruppe sein, wobei auch andere funktionelle Gruppen, beispielsweise Carboxygruppen, Epoxygruppen, Phosphatgruppen, Anhydride, Hydroxygruppen, Thiolgruppen und dergleichen sowie entsprechende Kombinationen möglich sind, die die kovalente Kopplung des Hydrogels an den optischen Teil erlauben. Beispielsweise kann die Silanverbindung 3-(Triethoxysilyl)propan-1-amin oder Triethoxy(hex-5-enyl)silan sein. Im Fall eines Aminogruppen tragenden Silans und eines Aminogruppen tragenden Hydrogels können zum kovalenten Koppeln Quervernetzer verwendet werden, die zwei oder mehr Carboxylgruppen tragen und optional zusätzlich eine oder mehrere (biologisch) abbaubare Bindungen enthalten. Über eine an sich bekannte EDC (1-Ethyl-3-(3-dimethylaminopropyl)carbodiimidhydrochlorid)/NHS (1-Hydroxy-2,5-pyrrolidindion) vermittelte Kopplungsreaktion kann das Hydrogel dann über Peptidbindungen kovalent mit dem optischen Teil gekoppelt werden. Die funktionellen Gruppen können natürlich auch vertauscht werden, das heißt dass das Hydrogel Carboxylgruppen und der Quervernetzer Aminogruppen tragen. Alternativ kann es vorgesehen sein, dass das Silan eine funktionelle Alkenylgruppe trägt, die über einen mindestens zwei Alkenylgruppen tragenden Quervernetzer (Dien, Trien etc.) mit einem Thiolgruppen tragenden Hydrogel über eine Reaktion vom Thiol-Michael-Typ gekoppelt wird. In Abhängigkeit der jeweils vorhandenen funktionellen Gruppen sind generell aber natürlich auch andere geeignete Vernetzungsreaktionen denkbar.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar. Es sind somit auch Ausführungen als offenbart anzusehen, die in der Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Darstellung eines Wirkstoffabgabesystems, welches eine Matrix aus einem biologisch abbaubaren Hydrogel mit eingelagerten Wirkstoffmolekülen umfasst, die durch Abbau des Hydrogels freigesetzt werden;
- Fig. 2: eine schematische Darstellung des Wirkstoffabgabesystems, welches eine Matrix aus einem nicht-abbaubaren Hydrogel mit eingelagerten Wirkstoffmolekülen umfasst, die durch Diffusion aus dem Hydrogel freigesetzt werden;
- Fig. 3: schematisch den Ablauf einer Kopplungsreaktion des biologisch abbaubaren Hydrogels an einen optischen Teil eines ophthalmologischen Implantats und einer Beladung des Hydrogels mit einem pharmakologischen Wirkstoff;
- Fig. 4: schematisch den Ablauf einer Kopplungsreaktion des nicht-abbaubaren Hydrogels an einen optischen Teil eines ophthalmologischen Implantats, wobei das Hydrogel über abbaubare Bindungen mit dem pharmakologischen Wirkstoff gekoppelt ist;
- Fig. 5: schematisch den Ablauf einer Freisetzung des im abbaubaren Hydrogel enthaltenen Wirkstoffs durch biologischen Abbau des Hydrogels; und
- Fig. 6: schematisch den Ablauf einer Freisetzung des im nicht-abbaubaren Hydrogel enthaltenen Wirkstoffs.

### Bevorzugte Ausführung der Erfindung

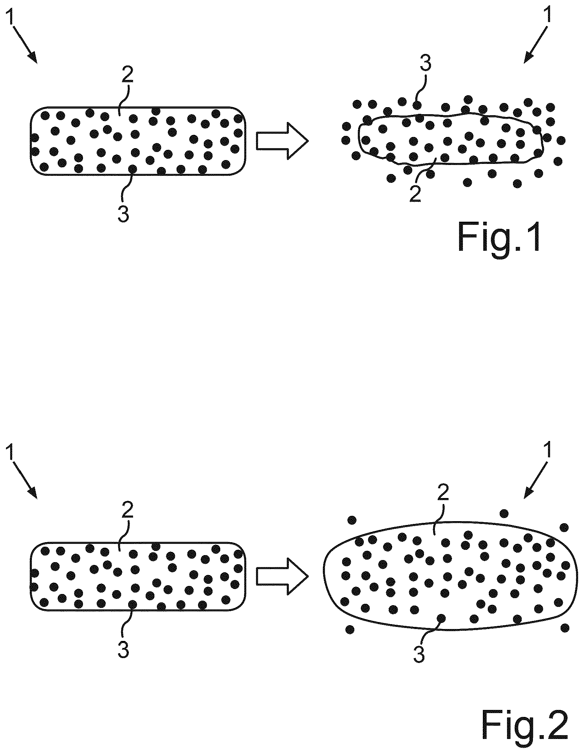

Fig. 1 zeigt eine schematische Darstellung eines Wirkstoffabgabesystems 1, welches eine Matrix aus einem biologisch abbaubaren Hydrogel 2 mit eingelagerten Wirkstoffmolekülen 3 umfasst, die durch Abbau des Hydrogels 2 in die Umgebung freigesetzt werden. Das Hydrogel 2 kann beispielsweise ausgewählt sein aus der Gruppe Poly(N-isopropylacrylamid), Poly(vinylalkohol), Poly(ethylenglykol), Hyaluronsäure, Cellulose, Poly(milchsäure) und dergleichen. Solche hydrophilen langkettigen Polymere können beispielsweise durch einfache Vernetzungsreaktionen zu Hydrogelen 2 umgewandelt werden. Aufgrund der großen Menge an hydrophilen Gruppen kann das Hydrogel 2 anschließend eine große Menge an Wasser aufnehmen, oft ein Vielfaches seines eigenen Ausgangs- bzw. Trockengewichts. Dieselben Wechselwirkungen können genutzt werden, um Wirkstoffmoleküle 3 innerhalb des Hydrogelsystems 2 physikalisch zu "fangen". Im vorliegenden Fall wird das Hydrogel 2 biologisch abgebaut, beispielsweise durch im Auge vorkommende Enzyme. Hierdurch werden die zuvor "gefangenen" Wirkstoffmoleküle 3 freigesetzt.

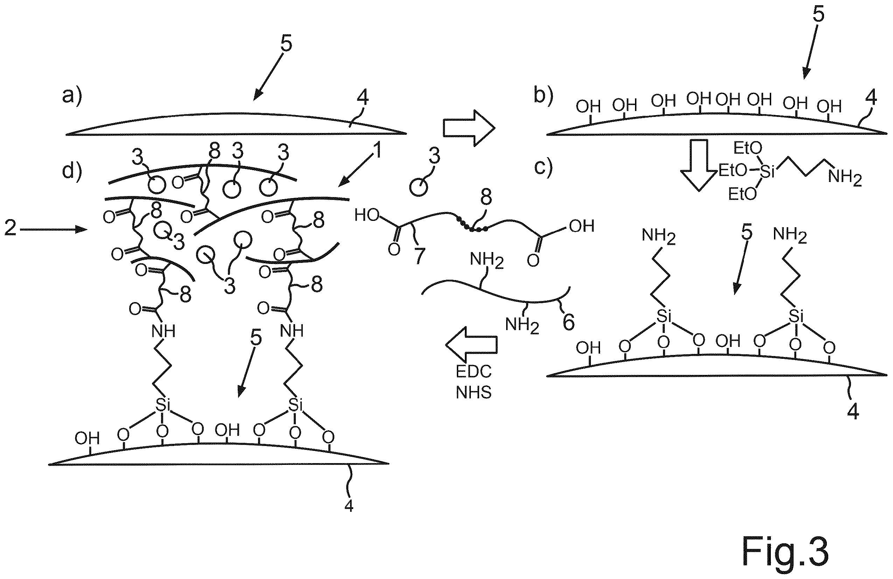

Fig. 2 zeigt eine schematische Darstellung des Wirkstoffabgabesystems 1, welches eine Matrix aus einem nicht-abbaubaren Hydrogel 2 mit eingelagerten Wirkstoffmolekülen 3 umfasst, die im Unterschied zu Fig. 1 durch Diffusion aus dem Hydrogel 2 freigesetzt werden. Man erkennt, dass das Hydrogel 2 durch Aufnahme von zusätzlichem Wasser aufquillt, wodurch die Porengröße des Polymernetzwerks vergrößert wird und die Diffusion der Wirkstoffmoleküle 3 erleichtert bzw. überhaupt ermöglicht. Für eine lang anhaltende, kontrollierte Freisetzung der Wirkstoffmoleküle 3 können hydrophile und möglichst voluminöse Wirkstoffmoleküle 3 verwendet werden, da sie nur langsam aus dem Hydrogel 2 diffundieren und damit die Wahrscheinlichkeit einer Berstfreisetzung kurze Zeit nach der Implantation eines zugeordneten ophthalmologischen Implantats 4 (s. Fig. 3) in ein Auge reduzieren. Kleine hydrophobe Wirkstoffmoleküle 3 können aber auch erfolgreich in solchen Polymernetzwerken eingeschlossen werden, beispielsweise durch Copolymerisation mit anderen Monomere bzw. Oligomeren oder durch Manipulation der Oberfläche des Hydrogels 2. Alternativ können die Wirkstoffmoleküle 3 auch über biologisch oder anderweitig abbaubare Bindungen kovalent mit dem Hydrogel 2 verbunden werden. Über die Geschwindigkeit der Degradation dieser abbaubaren Bindungen kann dann die Freisetzung der Wirkstoffmoleküle 3 wesentlich kontrolliert werden. Alle genannten Materialien und Techniken zur Herstellung von medikamentenbeladenen Hydrogelen 2 können verwendet werden, um die Oberflächen von optischen Teilen 5 und gegebenenfalls auch von haptischen Teilen (nicht gezeigt) ophthalmologischer Implantate 4 entsprechend zu modifizieren.

Um eine kovalente Vernetzung eines Hydrogels 2 mit der Oberfläche des Implantats 4 über eine chemische Reaktion zu erreichen, können verschiedene Techniken verwendet werden. Eine bevorzugte Möglichkeit stellt das Aufpfropfen von reaktiven Gruppen, z. B. von Epoxiden oder Thiolen, auf die Oberfläche des Implantats 4 dar. Während einer Polymerisation des Hydrogels 2 bzw. des Hydrogel-Precursors 6 reagieren Teile des Polymernetzwerks mit der modifizierten Oberfläche, wodurch die Immobilisierung des Hydrogels 2 erreicht wird.

Dies kann beispielsweise durch den Einsatz der sogenannten Klickchemie auf eine chemisch modifizierte Oberfläche des Implantats 4 erfolgen. Hierbei wird die Zieloberfläche des Implantats 4, die mit der Hydrogel-Schicht versehen werden soll, einem Plasma ausgesetzt, wodurch Hydroxygruppen gebildet werden. Diese können leicht zum Beispiel mit Triethoxyslianen mit unterschiedlichen reaktiven Gruppen reagieren, wodurch im Wesentlichen eine Oberfläche entsteht, die vollständig oder überwiegend mit solchen reaktiven Gruppen bedeckt ist. Je nach verwendetem Reaktionstyp können diese Gruppen dann während der Hydrogelsynthese direkt mit den Polymeren reagieren. Wenn beispielsweise eine EDC/NHS-vermittelte Amidkupplungsreaktion zur Vernetzung des Hydrogels 2 eingesetzt wird, würde das Pfropfen von entweder Carboxylgruppen oder Amingruppen es ermöglichen, das Hydrogel 2 gleichzeitig zu bilden und an der Oberfläche des Implantats 4 zu immobilisieren. Wenn alternativ eine Thiol-Michael-Reaktion verwendet wird, würde das Pfropfen von Thiolen auf die Oberfläche eine Immobilisierung des Hydrogels 2 ermöglichen. Pfropfreaktionen mit Triethoxysilanen ermöglichen die Einführung einer Vielzahl von reaktiven Gruppen auf der Oberfläche des Implantats 4 und ermöglichen eine kontrollierte Immobilisierung in Bezug auf die bei der Herstellung des Hydrogels 2 verwendeten Reaktionstypen und damit eine hohe Flexibilität zur Darstellung von immobilisierten, medikamenteneluierenden Hydrogelen 2. Dies bietet eine breite Palette von Möglichkeiten, wie man Intraokularlinsen und andere ophthalmologische Implantate 4 herstellen kann, die mit einen Wirkstoff 3 freisetzenden Hydrogelen 2 modifiziert sind.

Fig. 3 zeigt schematisch den Ablauf einer Kopplungsreaktion des biologisch abbaubaren Hydrogels 2 an einen optischen Teil 5 eines ophthalmologischen Implantats 4 und einer Beladung des Hydrogels 2 mit einem pharmakologischen Wirkstoff 3. Die Wirkstoffmoleküle 3 werden im gezeigten Beispiel physikalisch im Hydrogel 2 während der Bildung eingeschlossen. Dies wird erreicht, indem zunächst das Implantat 4 in Schritt a) bereitgestellt und in Schritt b) die Oberfläche des optischen Teils 5 des Implantats 4 im zu beschichtenden Bereich durch Plasmaaktivierung mit Hydroxygruppen versehen wird. Diese Hydroxygruppen werden anschließend reaktiven Silanen ausgesetzt, wobei vorliegend exemplarisch 3-(Triethoxysilyl)propan-1-amin verwendet wird. In dieser Ausführungsform werden damit Aminogruppen aufgepfropft. Es ist zu betonen, dass generell auch andere Silane und andere funktionelle Gruppen verwendet werden können, solange die erforderlichen Reaktionstypen miteinander kompatibel sind.

In einem nächsten Schritt c) wird eine Mischung aus einem Polymer-Grundgerüst oder Precursor 6 des Hydrogels 2 mit Aminogruppen, einem Vernetzer 7 mit zwei (oder mehr) Carboxylgruppen sowie optional mindestens einer abbaubare Bindung 8, dem Wirkstoff 3 und EDC/NHS auf die Oberfläche des optischen Teils 5 aufgebracht. Dies initiiert eine Peptidbildung zwischen dem Vernetzer 7, dem Precursor 6 und den oberflächenfixierten Aminogruppen, wodurch als Wirkstoffabgabesystem 1 das immobilisierte Hydrogel 2 gebildet und die Wirkstoffmoleküle 3 darin eingeschlossen werden. Für das Polymer bzw. den Precursor 6 selbst können beliebige geeignete Polymer-Grundgerüst (z. B. Poly(ethylenglykol), Hyaluronsäure, Cellulose, Alginat etc.) verwendet werden, sofern sie die erforderliche(n) reaktive(n) Gruppe(n) tragen oder entsprechend modifiziert werden können, um die erforderliche(n) reaktive(n) Gruppe(n) zu tragen. Wenn beispielsweise ein Kohlenhydrat verwendet wird, kann zu diesem Zweck eine ähnliche EDC/NHS-vermittelte Peptidkopplung verwendet werden. Alternativ können die funktionellen Gruppen umgekehrt werden, indem beispielsweise Carboxylfunktionen aufgepfropft werden und ein Vernetzer 7 mit zwei (oder mehr) Aminogruppen verwendet wird.

Der Abbau der optionalen abbaubaren Bindung 8 aus dem Vernetzer 7 kann beispielsweise durch Hydrolyse erfolgen. Hierzu können aktive Esterbindungen vorgesehen sein. Alternativ kann ein enzymatischer Abbau genutzt werden, beispielsweise durch den Einsatz von Hyaluronsäure im Polymergerüst des Hydrogels 2, das im implantierten Zustand des Implantats 4 durch Hyaluronidase abgebaut werden kann. Alternativ oder zusätzlich können Peptidbindungen vorgesehen sein, die durch Peptidasen gespalten werden können.

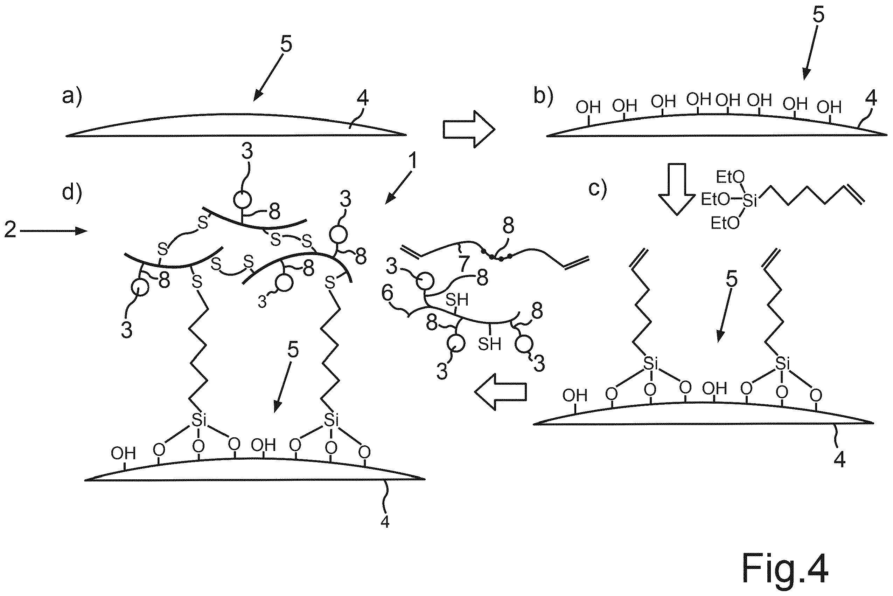

Fig. 4 zeigt schematisch den Ablauf einer Kopplungsreaktion des nicht-abbaubaren Hydrogels 2 an den optischen Teil 5 des ophthalmologischen Implantats 4, wobei das Hydrogel 2 über abbaubare Bindungen 8 mit dem pharmakologischen Wirkstoff 3 gekoppelt ist. In dieser exemplarischen Ausführungsform wird mit anderen Worten ein nicht (biologisch) abbaubares Hydrogel 2 verwendet. Dies geschieht nach einer Plasmaaktivierung in Schritt b) durch eine erste Pfropfung von Alkengruppen auf die Oberfläche des optischen Teils 5 in Schritt c) durch die bereits beschriebene Silanisierung. Als Silan wird dabei exemplarisch Triethoxy(hex-5-enyl)silan verwendet. In einem nächsten Schritt d) wird eine Mischung aus thiolgruppenhaltigen Polymeren bzw. Prescursoren 6 und Vernetzern 7 mit zwei (oder mehr) Alkengruppen auf die modifizierte Oberfläche aufgebracht. Dadurch wird eine Thiol-Michael-Reaktion zwischen den Alkenen und Thiolgruppen eingeleitet, die zu dem Wirkstoffabgabesystem 1 mit dem vernetzten, oberflächenimmobilisierten Hydrogel 2 und den eingelagerten Wirkstoffen 3 führt. Während die Wirkstoffe 3 im gezeigten Beispiel physikalisch im Hydrogel 2 eingeschlossen sind und anschließend über die Zeit durch Diffusion freigesetzt werden, können in einer alternativen Ausführungsform biologisch abbaubare Bindungen 8 vorgesehen sein, um die Wirkstoffmoleküle 3 kovalent mit dem Polymer-Grundgerüst des Hydrogels 2 zu verbinden. Dies ermöglicht eine bessere Kontrolle über das Freisetzungsprofil des Wirkstoffs 3 über die Abbaugeschwindigkeit dieser Bindungen 8. Dadurch können auch kleinere Wirkstoffmoleküle 3 über einen längeren Zeitraum ohne anfängliche Burst-Freisetzung freigesetzt werden. Der (biologische/hydrolytische) Abbau der Bindungen 8 zwischen dem Hydrogel 2 und dem Wirkstoff 3 bestimmt dabei das Freisetzungsprofil wesentlich.

Ähnlich wie bei der vorstehend beschriebenen Ausführungsform kann grundsätzlich jedes geeignete Polymer bzw. jeder geeignete Precursor 6 verwendet werden, der biokompatibel, mit den gewünschten chemischen Gruppen modifizierbar und in der Lage ist, Hydrogele 2 zu bilden. Insbesondere können alternative Ausgestaltungen der Klickchemie verwendet werden, um ein immobilisiertes Hydrogel 2 mit eingelagertem Wirkstoff 3 zu erhalten. Beide Ausführungsformen sind nur Beispiele dafür, wie medikamenteneluierende Hydrogele 2 als Wirkstoffabgabesystem 1 auf Oberflächen von ophthalmologischen Implantaten 4 hergestellt werden können und beschränken sich nicht auf die in diesen Beispielen gezeigten Reaktionstypen.

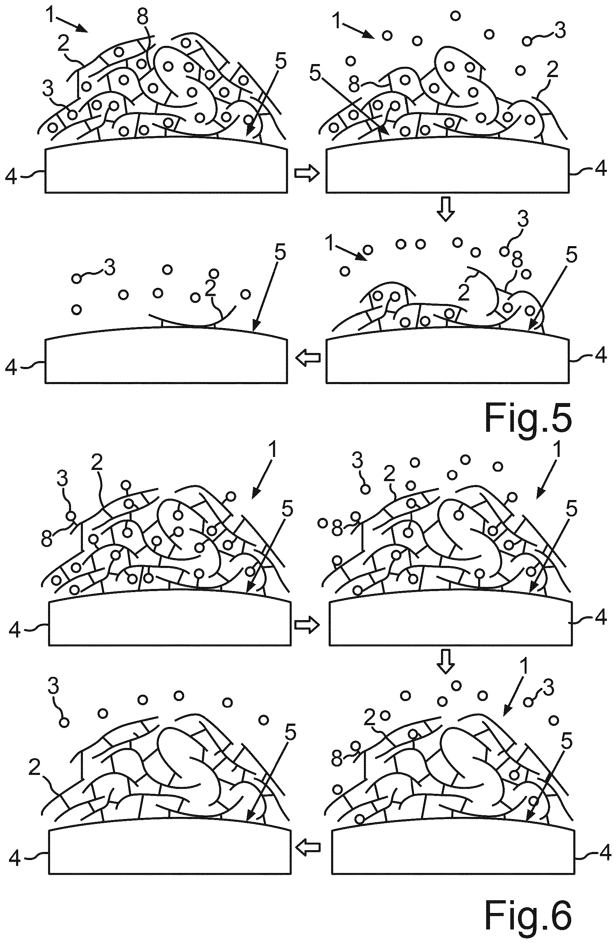

Fig. 5 zeigt schematisch den Ablauf einer Freisetzung des im abbaubaren Hydrogel 2 enthaltenen Wirkstoffs 3 durch biologischen Abbau der abbaubaren Verbindungen 8 des Hydrogels 2. Die Degradationsrate des Hydrogels 2 definiert dabei die Rate der Wirkstofffreisetzung des Wirkstoffabgabesystems 1.

Fig. 6 zeigt schematisch den Ablauf einer Freisetzung des im nicht-abbaubaren Hydrogel 2 enthaltenen Wirkstoffs 3. Die Wirkstoffmoleküle 3 sind dabei über abbaubare Verbindungen 8 an das nicht-abbaubare Polymergerüst des Hydrogels 2 gebunden. Nach dem Abbau der Verbindungen 8 erfolgt eine diffusionsbasierte Abgabe der Wirkstoffe 3, wodurch eine besonders lang anhaltende, gleichmäßige Abgabe ermöglicht ist.

Die Verwendung der beschriebenen Wirkstoffabgabesystemen 1 zur Beschichtung eines Teils oder der gesamten Oberfläche des optischen Teils 5 eines ophthalmologischen Implantats 4 erlaubt damit im Vergleich zu einer exklusiven Anordnung an einem haptischen Teil (nicht gezeigt) die Bereitstellung einer größeren Wirkstoffmenge, ohne die optische und haptische Funktionalität des Implantats 4 einzuschränken.

Ein weiterer Vorteil betrifft eine reduzierte Klebrigkeit der Oberfläche des Implantats 4. Bei manchen hydrophoben IOL-Materialien besteht das Problem, dass ihre Oberfläche vergleichsweise klebrig ist. Dies kann dazu führen, dass nach der chirurgischen Einführung in das Auge die IOL-Entfaltung ungünstig sein kann. Im Extremfall kann eine oder beide Haptiken an der IOL-Oberfläche des optischen Teils 5 haften bleiben, was eine zusätzliche Manipulation der IOL durch den Arzt erfordert. Diese Klebrigkeit kann dadurch vermieden werden, dass die Oberfläche mit mehreren Heparin- und Polyminschichten beschichtet wird. Allerdings gibt es mit diesem Ansatz eine Reihe von Problemen. Die Verwendung des Wirkstoffabgabesystems 1 zur teilweisen oder vollständigen Beschichtung des optischen Teils 5 löst auch das Problem der Klebrigkeit sowie einige der Probleme etablierter anderer Beschichtungen.

Ein weiterer vorteilhafter Aspekt des Wirkstoffabgabesystems 1 besteht in einer verbesserten Schmierung. Für die chirurgische Insertion werden Intraokularlinsen 4 gefaltet und mit einem Injektor in das Auge injiziert. Um eine mögliche Schädigung der Hornhaut durch Reduzierung der Schnittgröße zu verhindern, ist es wünschenswert, die Injektionsspitzen so klein wie möglich zu halten. Dies führt zu einer gewissen physischen Kraft, die aufgebracht werden muss, um die gefaltete IOL 4 durch den Injektor zu drücken. Diese Kräfte werden in der Regel durch eine Gleitbeschichtung der Innenwand des Injektors reduziert. Das immobilisierte Hydrogel 2 des Wirkstoffabgabesystems 1 kann dabei eine (zusätzliche) Schmierung bieten und entweder den Injektionsprozess verbessern oder die bislang notwendige Schmierung an der Injektorwand vollständig ersetzen.

Das Wirkstoffabgabesystem 1 kann auch zur PCO-Prävention verwendet werden. Eine häufige postoperative Komplikation der Kataraktoperation ist die "Post-operative Kapseltrübung" (PCO): Zellen wachsen auf der Kapselsack- und IOL-Oberfläche 5 und verursachen mit der Zeit einen Verschluss des Kapselsacks. Obwohl dies durch Laseranwendung behandelt werden kann, ist es dennoch wünschenswert, den Beginn von PCO so weit wie möglich zu verzögern oder ganz zu verhindern. Die vorstehend beschriebene Oberflächenmodifikation einer IOL 4 mit einem immobilisierten, Wirkstoffe 3 abgebenden Hydrogel 2 kann die PCO-Entwicklung verlangsamen oder vollständig verhindern. Darüber hinaus kann die Oberfläche des Hydrogels 2 optional so modifiziert werden, dass die PCO-Prävention zusätzlich unterstützt wird, z. B. durch das Aufpfropfen zusätzlicher funktioneller Gruppen an die Oberfläche des Hydrogels 2. Beispielsweise können COX-2 blockierende NSAIDS bei der Prävention von PCO helfen. Solche NSAID werden für die Behandlung von Entzündungen verwendet und können durch ihre Aufnahme in das Hydrogel 2 auch das Entstehen von PCO verhindern oder erheblich verlangsamen.

Das Wirkstoffabgabesystem 1 kann weiterhin als Plattform für unterschiedliche Medikamente und Wirkstoffe 3 verwendet werden. Neben der Verwendung von Entzündungshemmern als Wirkstoff 3 ist es durchaus möglich, das Wirkstoffabgabesystem 1 zur Aufnahme und Abgabe anderer Medikamententypen zu verwenden. Dazu könnten unter anderem Antibiotika zur Vorbeugung gegen bakterielle Infektionen oder Medikamente zur Verringerung des Augeninnendrucks gegen den Beginn des Glaukoms gehören. Wenn das Hydrogel 2 eine ausreichend großes Reservoir bietet, können auch zwei oder mehr Wirkstoffe 3 zur gleichzeitigen Langzeitfreisetzung verwendet werden.

Im Hinblick auf hydrophile IOLs und andere hydrophile ophthalmologische Implantate 4, die bis zur Implantation in Wasser gelagert werden, bieten sich verschiedene Strategien an, um eine hohe Lagerstabilität zu erreichen. Beispielsweise kann ein Implantat 4, das mit einem nicht (biologisch) abbaubaren Hydrogel 2 beschichtet ist, in einer gesättigten Wirkstofflösung gelagert werden. Auf diese Weise bleibt die Wirkstoffkonzentration innerhalb des Wirkstoffabgabesystems 1 während der Lagerung konstant.

Alternativ oder zusätzlich können abbaubare Bindungen 8 verwendet werden, die nicht hydrolyseempfindlich sind. Beispielsweise können Wirkstoffe 3 über abbaubare Peptidbindungen 8 an das Hydrogel 2 gebunden werden. Alternativ oder zusätzlich können auch Vernetzer 7, die Peptidbindungen enthalten und/oder Peptidbindungen erzeugen, verwendet werden. Peptidase-Enzyme in der Augenumgebung können dann nach der Implantation das Hydrogel 2 abbauen und/oder die Wirkstoffe 3 vom Polymergerüst des Hydrogels 2 trennen. Während der Lagerung sind keine Enzyme vorhanden, wodurch das Wirkstoffabgabesystem 1 lagerungsstabil ist.

Eine weitere Variante besteht in der Herstellung bzw. Verwendung eines Hydrogels 2, das die Diffusion der eingelagerten Wirkstoffe 3 stark einschränkt, z. B. durch kleine Porengröße und große Wirkstoffmoleküle 3. Das Hydrogel 2 kann aus einem enzymatisch abbaubaren Anteil, z. B. Hyaluronsäure, hergestellt sein. Während der Lagerung ist das Wirkstoffabgabesystem 1 stabil, da es erst im implantierten Zustand in der Augenumgebung mit Hyaluronidase (oder einem vergleichbaren Enzym) abgebaut wird und die eingelagerten Wirkstoffe 3 freigesetzt werden.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern, DIN-Toleranzen und dergleichen - als vom Rahmen der Erfindung mitumfasst anzusehen.

### Bezugszeichenliste

- 1: Wirkstoffabgabesystem
- 2: Hydrogel
- 3: Wirkstoff
- 4: Implantat
- 5: optischer Teil
- 6: Precursor
- 7: Vernetzer
- 8: abbaubare Bindung

## Patentansprüche

1. Ophthalmologisches Implantat (4) mit einem Wirkstoffabgabesystem (1), welches dazu ausgebildet ist, im implantierten Zustand des ophthalmologischen Implantats (4) wenigstens einen pharmakologischen Wirkstoff (3) abzugeben, wobei das Wirkstoffabgabesystem (1) wenigstens ein Hydrogel (2) als Matrix umfasst, wobei das Hydrogel (2) eine Schicht auf einem optischen Teil (5) des Implantats (4) bildet, kovalent an den optischen Teil (5) gebunden und mit dem wenigstens einen Wirkstoff (3) beladen ist,
**dadurch gekennzeichnet, dass**
der wenigstens eine Wirkstoff (3) in im Hydrogel (2) verteilten Polymernanopartikeln vorliegt.

2. Ophthalmologisches Implantat (4) nach Anspruch 1,
**dadurch gekennzeichnet, dass**
dieses als Intraokularlinse, insbesondere akkommodierende Intraokularlinse, ausgebildet ist.

3. Ophthalmologisches Implantat (4) nach Anspruch 1 oder 2,
**dadurch gekennzeichnet, dass**
das Hydrogel (2) abbaubar, insbesondere biologisch abbaubar ist.

4. Ophthalmologisches Implantat (4) nach einem der Ansprüche 1 bis 3,
**dadurch gekennzeichnet, dass**
das Hydrogel (2) ausgewählt ist aus einer Gruppe, die Poly(N-isopropylacrylamid), Polyvinylalkohol, Polyethylenglycol, Polymilchsäure, Polyethylenimin, Cellulose, Celluloseether mit Methyl- und/oder Ethyl- und/oder Propylgruppen, insbesondere Hydroxypropylmethylcellulose, Hydroxyethylmethylcellulose und/oder Methylcellulose, Glykosaminoglykane, insbesondere Hyaluronsäure, Chondroitinsulfat, Dermatansulfat, Heparin, Heparansulfat, Keratansulfat, Alginsäure, Polymannuronsäure, Polyguluronsäure, Polyglucuronsäure, Amylose, Amylopektin, Callose, Chitosan, Polygalactomannan, Dextran, Xanthan, und/oder eine Mischung und/oder ein physiologisch akzeptables Salz davon umfasst.

5. Ophthalmologisches Implantat (4) nach einem der Ansprüche 1 bis 4,
**dadurch gekennzeichnet, dass**
der wenigstens eine Wirkstoff (3) über einen vorzugsweise biologisch abbaubaren Bindungstyp (8) kovalent an das Hydrogel (2) gebunden ist und/oder kovalent an ein Monomer und/oder Oligomer gebunden ist und/oder verteilt im Hydrogel (2) vorliegt und/oder in biologisch abbaubaren, im Hydrogel (2) verteilten Polymernanopartikeln vorliegt.

6. Ophthalmologisches Implantat (4) nach einem der Ansprüche 1 bis 5,
**dadurch gekennzeichnet, dass**
wenigstens ein pharmakologischer Wirkstoff (3) kovalent auf zumindest einem Oberflächenbereich des Hydrogels (2) gebunden ist.

7. Ophthalmologisches Implantat (4) nach einem der Ansprüche 1 bis 6,
**dadurch gekennzeichnet, dass**
der wenigstens eine Wirkstoff (3) ausgewählt ist aus einer Gruppe, die steroidale und nicht-steroidale Entzündungshemmer, insbesondere COX-2-Hemmer, Prostaglandine und/oder Prostamide, Antibiotika und Betablocker umfasst.

8. Ophthalmologisches Implantat (4) nach einem der Ansprüche 1 bis 7,
**dadurch gekennzeichnet, dass**
dieses in einer gesättigten Lösung des wenigstens einen Wirkstoffs (3) gelagert ist.

9. Verfahren zum Herstellen eines ophthalmologischen Implantats (4) mit einem Wirkstoffabgabesystem (1), welches dazu ausgebildet ist, im implantierten Zustand des ophthalmologischen Implantats (4) wenigstens einen pharmakologischen Wirkstoff (3) abzugeben, wobei ein optischer Teil (5) des Implantats (4) mit wenigstens einem Hydrogel (2) als Matrix des Wirkstoffabgabesystems (1) beschichtet und das wenigstens eine Hydrogel (2) kovalent an den optischen Teil (5) gebunden wird, wobei das Hydrogel (2) mit dem wenigstens einen Wirkstoff (3) beladen wird,
**dadurch gekennzeichnet, dass**
der wenigstens eine Wirkstoff (3) in Polymernanopartikeln vorliegt, die im Hydrogel (2) verteilt werden.

10. Verfahren nach Anspruch 9,
**dadurch gekennzeichnet, dass**
das kovalente Binden des wenigstens einen Hydrogels (2) an den optischen Teil (5) des Implantats (4) die Schritte
- Erzeugen von oberflächlichen Hydroxygruppen auf dem optischen Teil (5) des Implantats (4);
- Pfropfpolymerisieren wenigstens einer reaktiven Silanverbindung, welche neben einer Silangruppe wenigstens eine weitere funktionelle Gruppe trägt, auf die oberflächlichen Hydroxygruppen des Implantats (4); und
- kovalentes Binden des wenigstens einen Hydrogels (2) an die weitere funktionelle Gruppe der aufgepfropften Silanverbindung
umfasst.

## Claims

1. Ophthalmological implant (4) having an active ingredient release system (1) designed to release at least one pharmacologically active ingredient (3) in the implanted state of the ophthalmological implant (4), where the active ingredient release system (1) comprises at least one hydrogel (2) as matrix, where the hydrogel (2) forms a layer on an optical component (5) of the implant (4), is covalently bonded to the optical component (5) and is laden with the at least one active ingredient (3),
**characterized in that**
the at least one active ingredient (3) is in the form of polymer nanoparticles distributed within the hydrogel (2) .

2. Ophthalmological implant (4) according to Claim 1,
**characterized in that**
it takes the form of an intraocular lens, especially an accommodating intraocular lens.

3. Ophthalmological implant (4) according to Claim 1 or 2,
**characterized in that**
the hydrogel (2) is degradable, especially biodegradable.

4. Ophthalmological implant (4) according to any of Claims 1 to 3,
**characterized in that**
the hydrogel (2) is selected from a group comprising poly(N-isopropylacrylamide), polyvinylalcohol, polyethyleneglycol, polylactic acid, polyethyleneimine, cellulose, cellulose ethers having methyl and/or ethyl and/or propyl groups, especially hydroxypropyl methylcellulose, hydroxyethyl methylcellulose and/or methylcellulose, glycosaminoglycans, especially hyaluronic acid, chondroitin sulfate, dermatan sulfate, heparin, heparan sulfate, keratan sulfate, alginic acid, polymannuronic acid, polyguluronic acid, polyglucuronic acid, amylose, amylopectin, callose, chitosan, polygalactomannan, dextran, xanthan and/or a mixture and/or a physiologically acceptable salt thereof.

5. Ophthalmological implant (4) according to any of Claims 1 to 4,
**characterized in that**
the at least one active ingredient (3) is covalently bonded to the hydrogel (2) via a preferably biodegradable type of bond (8) and/or is covalently bonded to a monomer and/or oligomer and/or is distributed in the hydrogel (2) and/or is in the form of biodegradable polymer nanoparticles distributed within the hydrogel (2).

6. Ophthalmological implant (4) according to any of Claims 1 to 5,
**characterized in that**
at least one pharmacologically active ingredient (3) is covalently bonded to at least one surface region of the hydrogel (2).

7. Ophthalmological implant (4) according to any of Claims 1 to 6,
**characterized in that**
the at least one active ingredient (3) is selected from a group comprising steroidal and non-steroidal inflammation inhibitors, especially COX-2 inhibitors, prostaglandins and/or prostamides, antibiotics and beta-blockers.

8. Ophthalmological implant (4) according to any of Claims 1 to 7,
**characterized in that**
it is stored in a saturated solution of the at least one active ingredient (3).

9. Process for producing an ophthalmological implant (4) having an active ingredient release system (1) designed to release at least one pharmacologically active ingredient (3) in the implanted state of the ophthalmological implant (4), where an optical component (5) of the implant (4) is coated with at least one hydrogel (2) as matrix of the active ingredient release system (1), and the at least one hydrogel (2) is covalently bonded to the optical component (5), where the hydrogel (2) is laden with the at least one active ingredient (3),
**characterized in that**
the at least one active ingredient (3) is in the form of polymer nanoparticles distributed within the hydrogel (2) .

10. Process according to Claim 9,
**characterized in that**
the covalent binding of the at least one hydrogel (2) to the optical component (5) of the implant (4) comprises the steps of
- generating surface hydroxyl groups on the optical component (5) of the implant (4);
- graft polymerizing at least one reactive silane compound bearing at least one further functional group as well as a silane group onto the surface hydroxyl groups of the implant (4); and
- covalently binding the at least one hydrogel (2) to the further functional group of the grafted silane compound.

## Revendications

1. Implant ophtalmologique (4) comportant un système de distribution (1) de principe actif, qui est conçu pour distribuer au moins un principe actif pharmacologique (3) dans l'état implanté de l'implant ophtalmologique (4), le système de distribution (1) de principe actif comprenant au moins un hydrogel (2) en tant que matrice, l'hydrogel (2) formant une couche sur une partie optique (5) de l'implant (4), étant lié par liaison covalente à la partie optique (5) et étant chargé avec ledit au moins un principe actif (3),
**caractérisé en ce que**
ledit au moins un principe actif (3) est présent dans des nanoparticules de polymère réparties dans l'hydrogel (2) .

2. Implant ophtalmologique (4) selon la revendication 1,
**caractérisé en ce que**
ce dernier est conçu sous forme de lentille intraoculaire, en particulier lentille intraoculaire accommodative.

3. Implant ophtalmologique (4) selon la revendication 1 ou 2,
**caractérisé en ce que**
l'hydrogel (2) est dégradable, en particulier biologiquement dégradable.

4. Implant ophtalmologique (4) selon l'une quelconque des revendications 1 à 3 ,
**caractérisé en ce que**
l'hydrogel (2) est choisi dans un groupe qui comprend le poly(N-isopropyl-acrylamide), le poly(alcool vinylique), le polyéthylèneglycol, le poly(acide lactique), la polyéthylèneimine, la cellulose, les éthers de cellulose à groupes méthyle et/ou éthyle et/ou propyle, en particulier l'hydroxypropylméthylcellulose, l'hydroxyéthylméthylcellulose et/ou la méthylcellulose, les glycosaminoglycanes, en particulier l'acide hyaluronique, le sulfate de chondroïtine, le sulfate de dermatane, l'héparine, le sulfate d'héparane, le sulfate de kératane, l'acide alginique, le poly(acide mannuronique), le poly(acide glucuronique), l'amylose, l'amylopectine, le callose, le chitosane, le polygalactomannane, le dextrane, le xanthane, et/ou un mélange et/ou un sel physiologiquement acceptable de ceux-ci.

5. Implant ophtalmologique (4) selon l'une quelconque des revendications 1 à 4 ,
**caractérisé en ce que**
ledit au moins un principe actif (3) est, par un type de liaison (8) de préférence biologiquement dégradable, lié par liaison covalente à l'hydrogel (2) et/ou lié par liaison covalente à un monomère et/ou oligomère et/ou est présent réparti dans l'hydrogel (2) et/ou est présent dans des nanoparticules de polymère biologiquement dégradables, réparties dans l'hydrogel (2).

6. Implant ophtalmologique (4) selon l'une quelconque des revendications 1 à 5,
**caractérisé en ce**
**qu'**au moins un principe actif pharmacologique (3) est lié par liaison covalente à au moins une zone superficielle de l'hydrogel (2).

7. Implant ophtalmologique (4) selon l'une quelconque des revendications 1 à 6 ,
**caractérisé en ce que**
ledit au moins un principe actif (3) est choisi dans un groupe qui comprend des anti-inflammatoires stéroïdiens et des anti-inflammatoires non stéroïdiens, en particulier les inhibiteurs de COX-2, les prostaglandines et/ou les prostamides, les antibiotiques et les bêtabloquants.

8. Implant ophtalmologique (4) selon l'une quelconque des revendications 1 à 7,
**caractérisé en ce que**
ce dernier est conservé dans une solution saturée dudit au moins une principe actif (3).

9. Procédé pour la fabrication d'un implant ophtalmologique (4) comportant un système de distribution (1) de principe actif qui est conçu pour distribuer au moins un principe actif pharmacologique (3) dans l'état implanté de l'implant ophtalmologique (4), dans lequel une partie optique (5) de l'implant (4) est revêtue avec au moins un hydrogel (2) en tant que matrice du système de distribution (1) de principe actif et ledit au moins un hydrogel (2) est lié par liaison covalente à la partie optique (5), l'hydrogel (2) étant chargé avec ledit au moins un principe actif (3),
**caractérisé en ce que**
ledit au moins un principe actif (3) est présent dans des nanoparticules de polymère qui sont réparties dans l'hydrogel (2) .

10. Procédé selon la revendication 9,
**caractérisé en ce que**
ladite liaison covalente dudit au moins un hydrogel (2) à la partie optique (5) de l'implant (4) comprend les étapes
- production de groupes hydroxy superficiels sur la partie optique (5) de l'implant (4) ;
- polymérisation par greffage d'au moins un composé silane réactif qui, outre un groupe silane, porte au moins un autre groupe fonctionnel, sur les groupes hydroxy superficiels de l'implant (4) ; et
- liaison covalente dudit au moins un hydrogel (2) à l'autre groupe fonctionnel du composé silane ajouté par greffage.
